# EUROPEAN PATENT SPECIFICATION

(11) **EP 2 514 822 B1**
(45) Date of publication and mention of the grant of the patent: **27.09.2017**
(21) Application number: 10837957.9
(22) Date of filing: 13.12.2010
(51) Int. Cl.: C12N 15/115, C12Q 1/68, A61K 31/7088

(54) **ANTI-THROMBOSIS APTAMERS AND METHOD FOR STABILIZING THE STRUCTURE THEREOF**
ANTI-THROMBOSE-APTAMERE UND VERFAHREN ZUR STABILISIERUNG DER STRUKTUR DAVON
APTAMÈRES ANTIMICROBIENS ET PROCÉDÉ DE STABILISATION DE LEUR STRUCTURE

(30) Priority: 15.12.2009 RU 2009146170
(43) Date of publication of application: 24.10.2012
(73) Proprietor: Limited Liability Company IRWIN 2, Moscow Region 140000 (RU)
(72) Inventor: GOLOVIN, Andrei Viktorovich, Moscow 117465 (RU); KOPYLOV, Alexei Mikhailovich, Moscow 119619 (RU); RESHETNIKOV, Roman Vladimirovich, Moscow 109457 (RU); ZAVYALOVA, Elena Gennadievna, Moscow 115211 (RU); PAVLOVA, Galina Valerievna, Moscow 117513 (RU); BABIY, Vladimir Evstakhievich, Moscow 117420 (RU)
(74) Representative: Hoffmann Eitle
(86) International application number: PCT/RU2010/000750
(87) International publication number: WO 2011/075004

(56) References cited:
- WO-A2-02/103051
- WO-A2-2008/126123
- RU-C2- 2 304 168
- US-A1- 2006 035 254
- CHRIS M. OLSEN ET AL: "Unfolding of G-Quadruplexes: Energetic, and Ion and Water Contributions of G-Quartet Stacking", THE JOURNAL OF PHYSICAL CHEMISTRY B, vol. 110, no. 13, 1 April 2006 (2006-04-01), pages 6962-6969, XP055090393, ISSN: 1520-6106, DOI: 10.1021/jp0574697
- SMIRNOV I ET AL: "Effect of Loop sequence and size on DNA aptamer stability", BIOCHEMISTRY, vol. 39, 1 January 2000 (2000-01-01), pages 1462-1468, XP008095840, ISSN: 0006-2960, DOI: 10.1021/BI9919044
- DONALD M. GRAY ET AL: "Measured and calculated CD spectra of G-quartets stacked with the same or opposite polarities", CHIRALITY, vol. 20, no. 3-4, March 2008 (2008-03), pages 431-440, XP055089925, ISSN: 0899-0042, DOI: 10.1002/chir.20455
- JAUMOT J ET AL: "Classification of nucleic acids structures by means of the chemometric analysis of circular dichroism spectra", ANALYTICA CHIMICA ACTA, vol. 642, no. 1-2, 29 May 2009 (2009-05-29), pages 117-126, XP026096357, ISSN: 0003-2670, DOI: 10.1016/J.ACA.2008.12.052
- KAZUNORI IKEBUKURO ET AL.: 'A novel method of screening thrombin - inhibiting DNA aptamers using an evolution - mimicking algorithm.' NUCLEIC ACIDS RESEARCH vol. 33, no. 12, 2005, pages E108, 1 - 7
- JUDITH M. HEALY ET AL.: 'Pharmacokinetics and Biodistribution of Novel Aptamer Compositions.' PHARMACEUTICAL RESEARCH vol. 21, no. 12, 2004, pages 2234 - 2246, XP019370664
- SPIRIDONOVA V.A. ET AL.: 'Aptamernye DNK - ingibittory trombina novogo tipa.' BIOORGANICHESKAYA KHIMIYA vol. 29, no. 5, 2003, pages 495 - 498
- B. GATTO ET AL.: 'Nucleic Acid Aptamers Based on the G-Quadruplex Structure: Therapeutic and Diagnostic Potential.' CURRENT MEDICINAL CHEMISTRY vol. 16, no. 10, 2009, pages 1248 - 1265, XP002634830
- VASILIOS M. ET AL.: 'Structures of the potassium - saturated, 2: 1, and intermediate,1 : 1, forms of a quadruplex DNA.' NUCLEIC ACIDS RESEARCH vol. 28, no. 9, 2000, pages 1969 - 1977
- PETER SCHULTZE ET AL.: 'Three-dimensional Solution Structure of the Thrombinbinding DNA Aptamer d (GGTTGGTGTGGTTGG)' J. MOL. BIOL. vol. 235, 1994, pages 1532 - 1547, XP024008599

## Description

### Field of the invention

The present invention relates to the field of biotechnology and medicine, in particular, to a two-quadruplex DNA aptamer for use in a method of preventing and/or treating a condition associated with an undesirable thrombin activity. Further disclosed is
a method for stabilizing anti-thrombosis aptamers and to the production of novel stable anti-thrombosis aptamers having anticoagulant properties and anti-thrombotic action, wherein the aptamers can be used as anticoagulant and anti-thrombotic therapeutic agents.

### Background

Blood clotting (hemocoagulation, coagulation) is a part of homeostasis, preventing the loss of blood from an injured vessel by formation of a blood clot (thrombus) in the blood vessel.

Coagulation is a result of a complex series of enzymatic reactions. One of the most important steps is a series of reactions of conversion of the proenzyme, prothrombin, into the active enzyme, thrombin.

Thrombin is multifunctional serine protease that has both procoagulant and anticoagulant activities. As procoagulant enzyme, thrombin activates fibrinogen, platelets and blood coagulation factors V, VIII and XIII. Thrombin specifically cleaves fibrinogen by initiating the polymerization of fibrin monomers, which is the first event in the process of blood coagulation. In addition, thrombin participates in the main event of the blood coagulation, in particular in the formation of a blood clot, since it activates platelet aggregation. Thus, due to its procoagulant activity, thrombin plays a key role in stopping bleeding (physiological homeostasis) and in vasoocclusive thrombus formation (pathological thrombosis). Thrombin exhibits its anticoagulant activity when binding to thrombomodulin, glycoprotein that is expressed on the surface of endothelial cells of blood vessels. Thrombomodulin bound to thrombin loses its ability to bind to fibrinogen and platelets and binds to protein C. The activation of protein C results in inhibition of coagulation due to the proteolytic degradation of coagulation factors Va and VIIIa. Thus, formation of the thrombin-thrombomodulin complex shifts the thrombin activity from procoagulant to anticoagulant. The balance between the both activities is a key factor in the homeostasis regulation.

Blood vessel injury and thrombus formation are important factors in the pathogenesis of various vessel diseases, including atherosclerosis. Pathological activation of the blood coagulation system leads to thrombosis in various diseases.

Given the important role of thrombin in the blood clotting process, it can be stated that thrombin inhibitors, or anti-thrombosis agents, are extremely important for the prevention or treatment of thrombosis.

Currently, the most popular and widely used anti-thrombosis agent is heparin, which is not a direct thrombin inhibitor, and enhances the inhibitory action of antithrombin. The use of heparin is associated with multiple side effects, including bleeding and thrombocytopenia (low platelet counts).

In addition, the use of heparin is associated with adverse reactions such as:
- nonspecific binding to plasma proteins, leading to heparin resistance in patients;
- heparin cannot inhibit thrombin in a thrombus; heparin has nonlinear kinetics, making it impossible to control dosage;
- heparin is derived from animal tissues (bovine and porcine heparin), which can cause a risk of transmitting viruses and/or prions.

It should be especially noted that heparin is not effective in disseminated intravascular coagulation patients having a low plasma antithrombin activity, for example, lower than about 80% (Kenji Okajima, "Hanshusei kekkannai gyoko shokogun to tazoki fuzen (Disseminated intravascular coagulation syndrome and multiple organ failure)", lyaku (Medicine and Drug) Journal Co., Ltd.).

In recent years, a large number of highly effective anticoagulants, such as low-molecular-weight heparins, have been developed. However, these new anticoagulants have similar side effects: uncontrolled action, platelet activation, thrombocytopenia, paradoxical thrombosis and the like. The problem of disseminated intravascular coagulation patients with a low plasma antithrombin activity is still not solved. Low-molecular-weight heparin is also not a direct thrombin inhibitor. Its effect on thrombin is mediated by the factors of homeostasis.

In addition, in contrast to the indirect thrombin inhibitors, the antithrombotic effect of direct thrombin inhibitors does not depend on the presence of antithrombin III in plasma; platelet factor 4 and liver heparinase do not change their activity; they do not bind to plasma proteins, prevent thrombin-induced platelet activation, effectively inactivate clot-bound thrombin, i.e. have a more selective effect on thrombus formation.

A completely new approach to the development of thrombin inhibitors is to design of anti-thrombosis aptamers.

One of the first anti-thrombosis aptamers was obtained by SELEX method (Bock L.C., Griffin L.C., Latham J.A., Vermaas E.H., Toole J.J. Selection of single-stranded DNA molecules that bind and inhibit human thrombin. Nature, 1992 Feb 6, 355(6360): 564-6). The cited reference describes a DNA aptamer consisting of 15 nucleotides (dGGTTGGTGTGGTTGG), also called 15TGT, which specifically binds thrombin. Schultze P. et al., in the article "Three-dimensional solution structure of the thrombin-binding DNA aptamer d (GGTTGGTGTGGTTGG)", J. Mol. Biol. 1994 Feb 4; 235(5): 1532-47, has been studied the tertiary structure of a thrombin aptamer. ¹H NMR study has showed that the oligonucleotide forms a structure consisting of two guanine quartets connected by two lateral T-T loops and the T-G-T loop. The three-dimensional structure of the thrombin aptamer may be relevant for design of thrombin-inhibiting anticoagulants with similar structural motifs.

It is known that potassium ions stabilize the G-quadruplex structure (Besik I., Kankia et al., Folding of the Thrombin Aptamer into G-Quadruplex with Sr2+: Stability, Heat, and Hydration, J. Am. Chem. Soc., 2001, 123(44), pp.10799-10804, October 13, 2001); the complete folding of the structure occurs at high concentrations of potassium ions, whereas the physiological concentration is low and, as a rule, is only of 5 millimoles/liter (5 mM).

The publication of Nagatoishi (Nagatoishi S. et al., "Circular dichroism spectra demonstrate formation of the thrombin-binding DNA aptamer G-quadruplex under stabilizing-cation-deficient conditions", Biochem. Biophys. Res. Commun., 2007 Jan. 19; 352(3): 812-7. Epub 2006 Nov. 27) describes the possibility in principle of obtaining a stable thrombin aptamer at a low concentration of potassium ions.

In the review, Gatto B. (Gatto B. et al., "Nucleic acid aptamers based on the G-quadruplex structure: herapeutic and diagnostic potential", Curr. Med. Chem. 2009, 16(10):1248-65) assumed that a thrombin aptamer can be used for medical purposes, in particular, for diagnosis and treatment of blood diseases.

Griffin L.C., in the article (Griffin L.C. et al., "In vivo anticoagulant properties of a novel nucleotide-based thrombin inhibitor and demonstration of regional anticoagulation in extracorporeal circuits", Blood, 1993 Jun. 15; 81(12):3271-6) described an effort to use in vivo an unstable thrombin aptamer.

Despite the fact that the obtained DNA aptamer is a direct thrombin inhibitor, it has a low activity and a short lifetime in the blood due to the loss of its tertiary structure necessary to interact with thrombin, even at 30°C.

Thus, the main problem in further use of the anti-thrombosis aptamer in mammal medicine, including humans, is its extreme structural instability, and as a result - its minimal (poor) efficacy in vivo.

Thus, in this field there is still a necessity to design stable thrombin inhibitors that would be useful as therapeutic agents for the treatment of diseases associated with coagulation and that would provide a quick and controlled response without side effects.

### The essence invention

The present inventors have surprisingly found that the upper quartet adjacent to the lateral TGT loop can be stabilized by stacking interactions, significantly increasing thereby the stability of the anti-thrombosis aptamer and, therefore, increasing the efficacy thereof.

On the basis of data known in the art, it is clear that the stabilization is possible by adding a double-stranded element to maintain the aptamer ends close together. However, the efficacy of this approach has not been confirmed experimentally (see the Japanese reference). In introducing a double-stranded element, the aptamer activity was varied within a very wide range.

Novel stable anti-thrombosis aptamers and a method of stabilizing quadruplex-containing aptamers have been developed on the basis of this phenomenon.

The inventors have found that the stability of the anti-thrombosis aptamer may increase when the stacking interactions takes place along with increasing the number of hydrogen bonds in the molecule by maintaining the geometry of the upper quartet of a quadruplex.

The most unexpected contribution to the stability is, as was found by the authors, the blocking of a cation in the quadruplex cavity. The blocking of a cation in the quadruplex cavity leads to the retardation of ion-exchange with the environment, making thereby it possible to use the anti-thrombosis aptamer under physiological conditions for therapeutic purposes.

Stabilization of the quadruplex may occur for two reasons: firstly, due to increasing stacking interactions per se, including an effect of shielding the heterocyclic bases of the upper quartet from water. Secondly, the stacking interactions restrict the mobility of the ion by fixing and stabilizing the ion in the quadruplex center.

As regards the first cause, it should be noted that eight hydrogen bonds are formed during formation of the quartet. Naturally, the hydrogen bonds between guanines can be replaced with hydrogen bonds with water molecules, destabilizing the quadruplex structure. At the same time, making such replacement difficult, it is possible to stabilize the structure.

In addition, a cation may be blocked inside the quadruplex not only by means of stacking interactions, but also by providing a steric hindrance to the migration of the ion due to introducing additional chemical groups which size is comparable with the quartet size. Such blocking groups can be covalently or non-covalently attached to the lateral TGT loop. Alternative or additional location of said chemical group may be a covalent or non-covalent connection with 5'-and 3'-ends of the oligonucleotide.

### Summary of the invention

The present invention relates to DNA aptamers that specifically bind to thrombin and block the activity thereof.

Thus, the present invention relates to anti-thrombosis aptamers, in particular, the present invention relates to aptamers with the following primary structure:
- two-quadruplex DNA aptamer (DTI-33) GGTTGGTGTGGNTGG-Tx-GGTTGGTGTGGNTGG,
where N is any nucleotide selected from A, C, T, G; lower indexes x, y, z are the number of nucleotides, wherein x, y, z are not equal to each other; Tx is "x" consecutive residues T; R is a purine nucleotide base.

Specifically, the present invention relates to a two-quadruplex DNA aptamer for use in a method of preventing or treating a condition associated with an undesirable thrombin activity, wherein the aptamer is an oligodeoxyribonucleotide with a nucleotide sequence of the general formula of: GGTTGGTGTGGNTGG-Tx-GGTTGGTGTGGNTGG; where Tx is one or more bases T, as defined in claim 1. Furthermore, the present invention relates to a two-quadruplex DNA aptamer for use in preventing and treating a condition associated with an undesirable thrombin activity, wherein the aptamer is an oligodeoxyribonucleotide with a nucleotide sequence of the general formula of: GGTTGGTGTGGNTGG-Tx-GGTTGGTGTGGNTGG; where Tx is one or more bases T, as defined in claim 8. Preferred embodiments of the present invention are provided according to the dependent claims.

Further disclosed are aptamers with the following primary structure:
- one-modular enhanced DNA aptamer (DTI-G84) NₓGGGTTGGGTGTGGGNTGGGN_{y};
- one-modular enhanced DNA aptamer (DTI-A6) NₓGTAGGTTGGTGNGGNTGGGGCN_{y};
- bimodular DNA aptamer with a modification of the lateral loop (DTI-P91) NₓGTAGGTTGGTGGGGNTGGRRCNₓ;
- tetramodular DNA aptamer (DTI-r91) TNₓGTAGGTTGGTGGGGNTGGRRCNₓAGN_{y}GTAGGTTGGTGGGGNTGGRRCN_{y}C;
- tetramodular DNA aptamer (DTI-Fx7) NₓCTAGGTTGGATGGGNTGGTGN_{z}GTAGGTTGGATGGGNTGGTCN_{y},
where N is any nucleotide selected from A, C, T, G; lower indexes x, y, z are the number of nucleotides, wherein x, y, z are not equal to each other; Tx is "x" consecutive residues T; R is a purine nucleotide base.

In another embodiment, the present disclosure relates to a method of stabilizing the structure of a DNA aptamer, which basic substructure consists of quadruplex and two lateral TT loops and which possesses a specific and high-affinity thrombin binding capacity, wherein the method provides the stabilization of the quadruplex by forming an additional stacking-interaction system by means of heterocyclic bases or analogues thereof.

In another embodiment, the present disclosure relates to a pharmaceutical composition comprising the aptamers according to the disclosure. In particular, the present disclosure relates to a composition comprising the anti-thrombosis aptamers according to the disclosure in combination with a pharmaceutically acceptable carrier.

In another embodiment, the present disclosure relates to methods of using the anti-thrombosis aptamers according to the present disclosure.
In still another embodiment, the present disclosure relates to a method of inhibiting thrombin by the anti-thrombosis aptamers according to the present disclosure.

In another embodiment, the present disclosure relates to the prevention or treatment of conditions associated with an undesirable thrombin activity. In particular, the present disclosure relates to the treatment of thrombosis by administering the anti-thrombosis aptamers according to the present invention.

In still another rembodiment, the present disclosure relates to diagnostic methods, wherein the method involves the use of the anti-thrombosis aptamers, in particular, to methods for diagnosing the level of thrombin, for example, in the form of diagnostic strips.

The present invention is described in more detail below with the references to the accompanied drawings for clarity and to specific embodiments that are not intended to limit the claimed invention.

### Brief description of the drawings

Fig.1 shows the dependence of thrombin time on the concentration of aptamer in human plasma. 31TGT is a reference aptamer described in literature (A novel method of screening thrombin-inhibiting DNA aptamers using an evolution-mimicking algorithm. Ikebukuro K., Okumura Y., Sumikura K., Karabe I. Nucleic Acids Res., 2005 Jul 7; 33(12):e108.), 15TCT is a derivative of the standard aptamer 15TGT. DPI-P91 is an aptamer stabilized according to the method of the present disclosure.

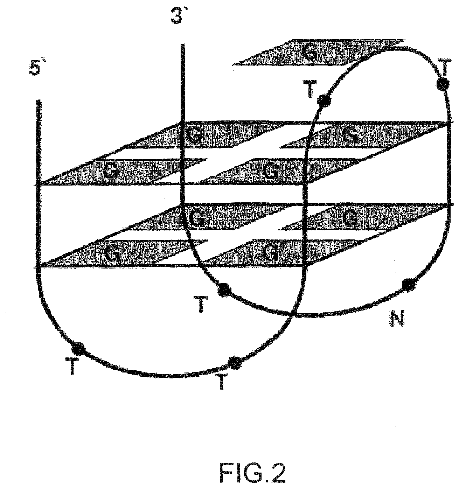

Fig.2 is schematic representation of the spatial structure of the anti-thrombosis aptamer 15TGT described in literature, interacting with thrombin. Heterocyclic bases G1, G6, G10, G15 form the upper quartet of the quadruplex. Heterocyclic base G8 is in stacking-interaction with the upper quartet.
Fig.3 shows a computer model of the spatial structure of the aptamer DPI-R91 stabilized according to the present disclosure. Bases G7 and G8 of the proposed structure and new bases G and A (shown in black) create a large surface of stacking-interaction with the upper quartet.
Fig.4 shows atom mobility (fluctuations) in the spatial structure of aptamers according to the data of computer molecular dynamics of two anti-thrombosis aptamers. The data that pertain to the claimed aptamer stabilized according to the patentable principles are shown in gray color. Data that pertain to aptamer 31TGT are shown in black color.

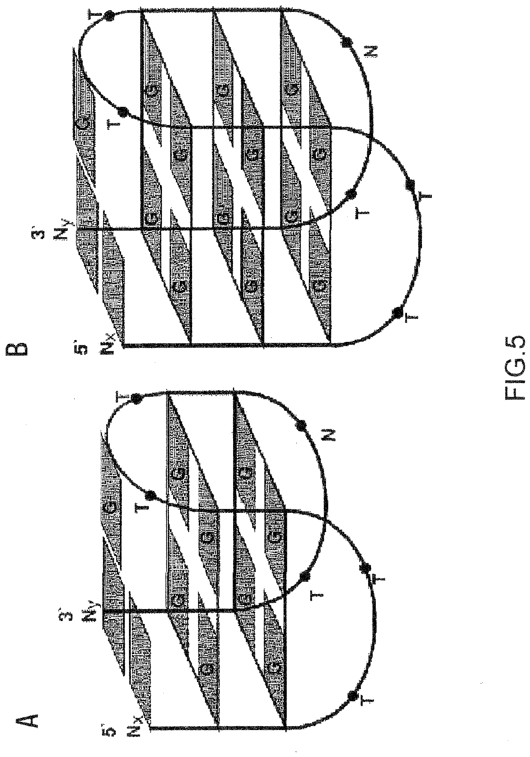

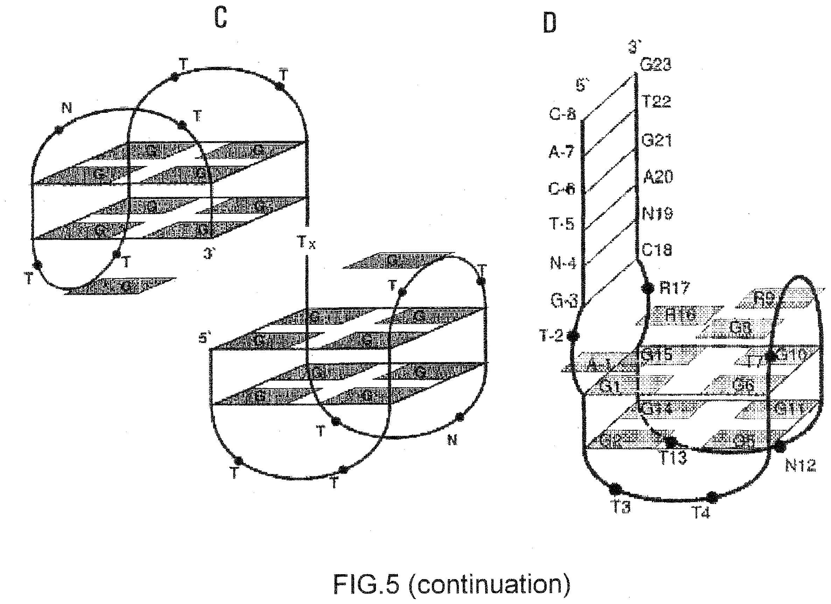

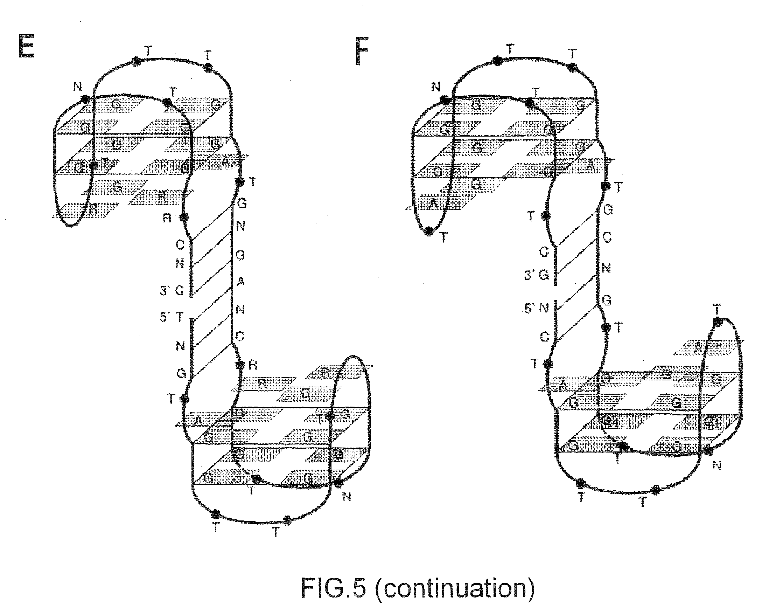

Fig.5 is schematic representation of the spatial structure of the claimed G-quadruplex variants that interact with thrombin and that are stabilized according to the present disclosure. A) Schematic representation of the aptamer DTI-A6. B) Schematic representation of the aptamer DTI-G8. C) Schematic representation of the aptamer DTI-33. D) Schematic representation of the aptamer DTI-P91. E) Schematic representation of the aptamer DTI-r91. F) Schematic representation of the aptamer DTI-Fx7.
Fig.6 shows the dependence of prothrombin time on the concentration of different aptamer DNAs in human blood plasma.
Fig.7 shows the dependence of thrombin time on the concentration of different aptamer DNAs in human blood plasma.
Fig.8 is schematic representation of the complex of thrombin (brown) with its substrate, fibrinogen; schematic representation of the formation of fibrin strands from fibrin monomers.
Fig.9A-B shows the inhibition of thrombin by the aptamer DTI-33 and by the aptamer 15TVA.
Fig. 10 shows the dependence of the aptamer affinity to thrombin at the initial concentration of thrombin.

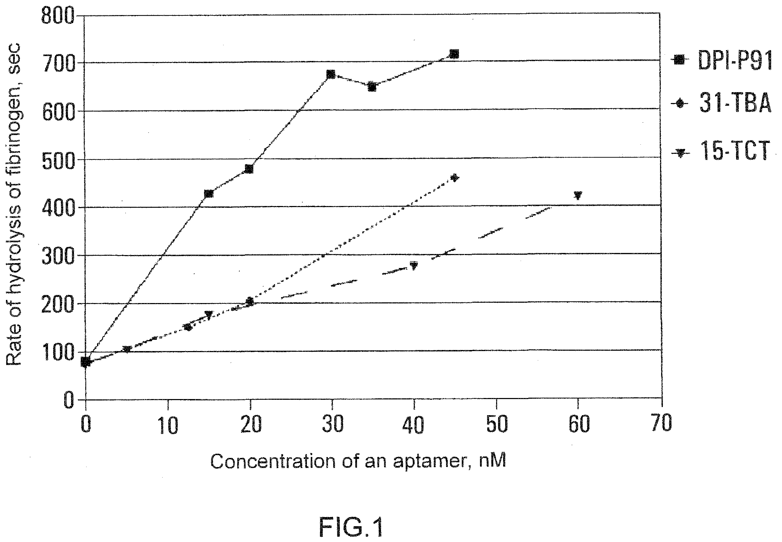

Fig.1 demonstrates study of the aptamer inhibitory property and stability in rats.

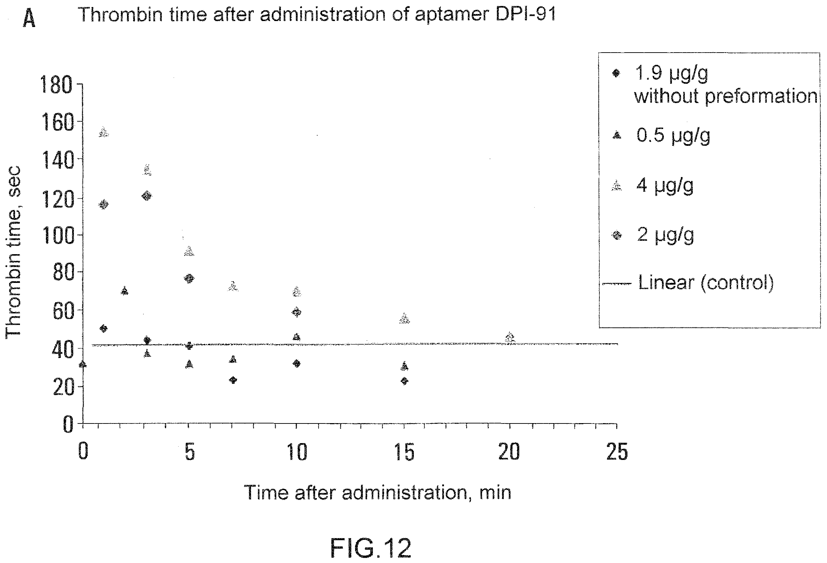

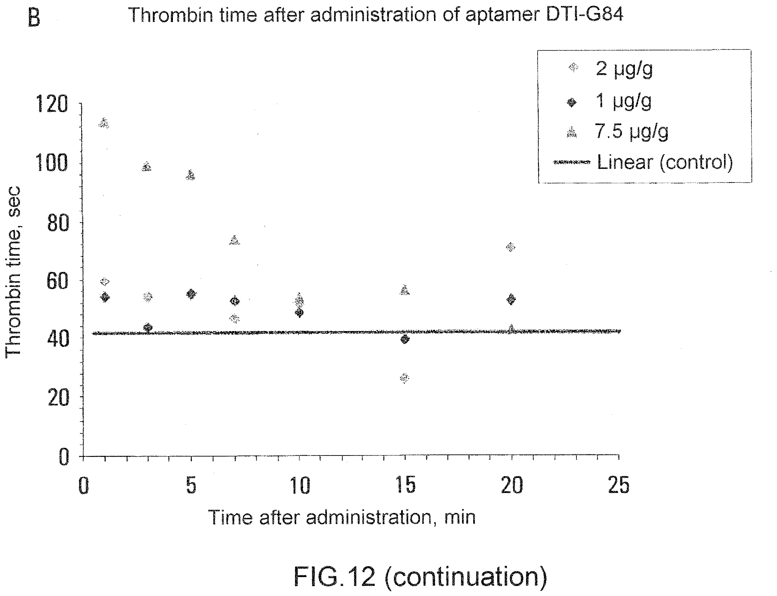

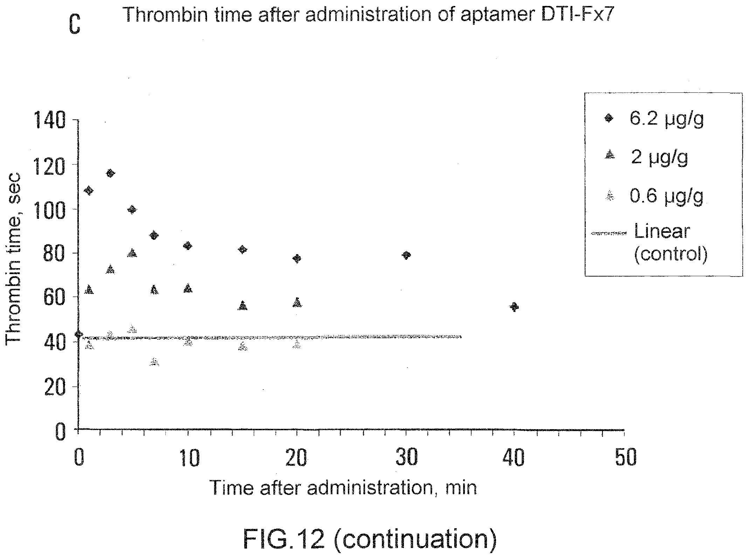

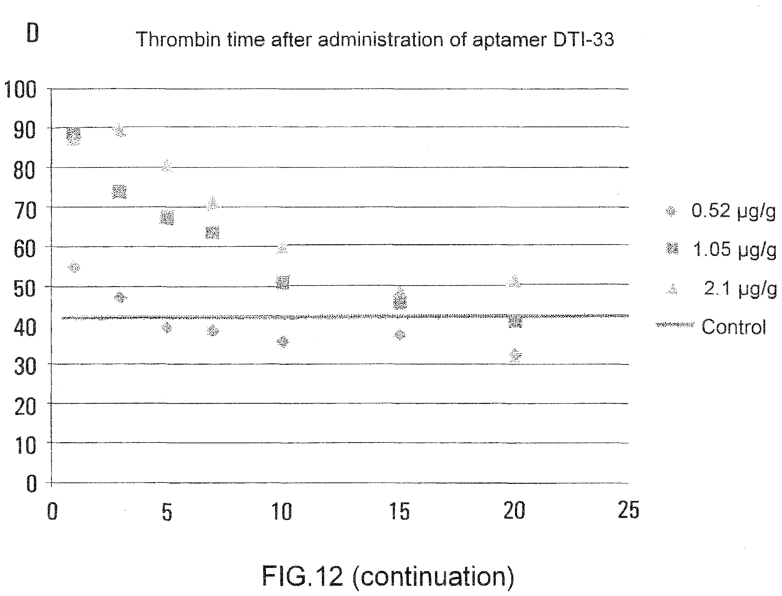

Fig.12A-D shows thrombin time after administration of the aptamer DPI-91; thrombin time after administration of the aptamer DTI-G84; thrombin time after administration of the aptamer DTI-Fx7; thrombin time after administration of the aptamer DTI-33.

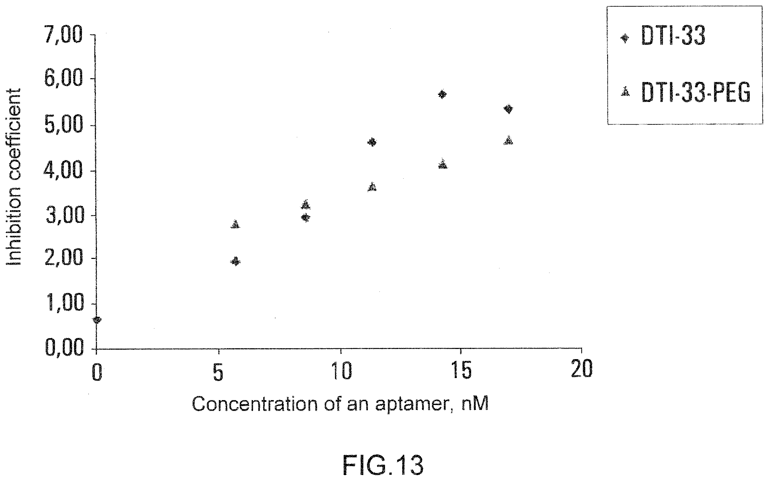

Fig.13 shows that chemical modifications of the aptamers do not lead to loss of the inhibitory activity thereof.

### Detailed description of the invention

The technical object of the present invention is to develop new methods of stabilizing the structure of anti-thrombosis aptamers due to their structure, and methods of obtaining the stable structures of the aptamers.

The technical object is achieved by searching for structural factors stabilizing the G-quadruplexes of anti-thrombosis aptamers by means of methods of molecular modeling and by implementing thereof in specific spatial structures. A rational design of modified aptamers that can effectively bind to thrombin is carried out on the basis of the revealed factors. Modified stable structures are prepared by direct chemical synthesis and their efficacy in regard to thrombin inhibition is compared with that of known aptamers.

The technical result of the claimed invention is to improve the efficacy of assembling anti-thrombosis aptamers and to increase the stability of the structure thereof under physiological conditions. Factors that determine positive effects for stabilization of anti-thrombosis DNA aptamers can be used for other G-quadruplex structures, including aptamers against other targets and telomere structures.

The present invention relates to DNA aptamers that specifically bind to thrombin and inhibit the formation of blood clots. The sequence of the DNA aptamers was selected by the iterative process of the systematic evolution of ligands by exponential enrichment (SELEX). In this process, a set of random oligonucleotide sequences (for example, with a size of about 10⁵ to about 10¹⁵) is bound to a target protein or to another target of interest; unbound oligonucleotides are removed, and the bound oligonucleotides are amplified and bound again. The process is repeated several times. As a result, a library comprising numerous sequences capable of binding to a selected target is produced. In one of embodiments, the aptamers can be selected from the sequences and according to specific requirements for the aptamer structure.
The length of the aptamers according to the invention is preferably of from about 20 to 80 nucleotides, in particular, from about 30 to about 50 nucleotides. In some embodiments of the present disclosure, aptamers may also include additional nucleotides at their 5'- and/or 3'-end. The additional nucleotides may be, for example, a part of primer sequences, sequences recognized by restriction enzymes, or the sequences of vectors that are used to determine individual aptamer sequences.

The term "aptamers" refers to oligonucleotides or peptide molecules that specifically bind to a target molecule. The target molecule can be either the whole cells, or low-molecular-weight organic compounds. Oligonucleotide aptamers with desired properties are isolated from random-sequence libraries by in vitro selection methods (SELEX) according to their capacity to interact specifically with corresponding immobilized targets/ligands. Aptamers are often chemically modified to improve their functional properties. Corresponding oligodeoxyribonucleotides were obtained by a conventional solid-phase synthesis method.

The term "duplex site" refers to the site, in a molecule, that forms a canonical double-stranded DNA helix.

The term "G-quadruplex" refers to guanine-rich nucleotide sequences which may form four-stranded structures (also known as G-tetrads, or C4-DNA). They consist of guanines arranged in a square-like configuration (quartets) stabilized by Hoogsteen hydrogen bonds. They also can be stabilized by a cation. Quadruplexes may be formed by DNA, RNA, LNA and PNA, and may be monomolecular, bimolecular, or tetramolecular. Depending on the direction (3'-5' or 5'-3') of the quartet-forming chains, the structures may be described as parallel or antiparallel. G-quadruplexes exhibit a characteristic circular dichroism spectrum.

The term "anti-thrombosis aptamer" refers to a minimum monomolecular quadruplex that is connected by three lateral loops. Under physiological conditions the anti-thrombosis aptamer loses tertiary quadruplex structure even at 25°C, which is far below physiological temperatures, for example human body temperature. In addition, since potassium ions stabilize the aptamer structure and the complete self-assembly of the structure occurs at high concentrations of potassium ions, at physiological potassium concentrations, which is 5 mmol/L (5 mM), the anti-thrombosis aptamer is unstable. Due to significant ionic exchange with the medium, the administration of an ion-stabilized aptamer to the blood of a mammal, in particular a human, will lead to the loss of its structure required for binding to thrombin. The thrombin-binding lateral T-T loops have a quadruplex-destabilizing effect. This fact points out that the T-T loop structure cannot be changed. The only area to be modified is the lateral TGT loop and 3'- and 5'-ends.

As used herein, the term "lower quartet" refers to a quartet adjacent to the lateral T-T loops, and the term "upper quartet" refers to a quartet adjacent to the TGT lateral loops.

The term "aptamer 31 TGT" refers to the reference aptamer, as described in literature (see, A novel method of screening thrombin-inhibiting DNA aptamers using an evolution-mimicking algorithm. Ikebukuro K., Okumura Y., Sumikura K., Karube I. Nucleic Acids Res., 2005 Jul. 7, 33(12):e108.) This is a variant of the anti-thrombosis aptamer with a duplex region to stabilize the ends.

The term "aptamer 15TST" refers to a derivative of the anti-thrombosis aptamer 15TGT, which is used as a negative control. The replacement of guanine by cytosine in the lateral TGT loop obviously weakens stacking-interactions since the surface of the aromatic group in the cytosine base is smaller than in guanine.

As used herein, the term "stacking" or "stacking interaction" refers to such an arrangement of aromatic molecules that are like the arrangement of coins in a pile and is supported by the interactions between the aromatic groups. The interplanar distance between the centers of mass of the atoms belonging to the aromatic groups is of 3.4 to 3.8 Å. A significant contribution of minimization of the hydrophobic surface also occurs along with the interaction between the aromatic groups in aqueous solutions. In case of aromatic compounds, such contribution may significantly increase the stability of a system.

In DNA, stacking interaction occurs between adjacent nucleotides and increases the stability of the molecular structure. The nitrogenous bases of a nucleotide consist of either purine or pyrimidine rings that are aromatic. In a DNA molecule, aromatic rings are mutually parallel, which allows the interaction between the bases. This type of the interaction is non-covalent. Although a non-covalent bond is weaker than a covalent bond, the total stacking interaction in a DNA molecule provides powerful stabilization.

"Aptamer sequence" used herein refers to a polymer sequence of nucleotides and derivatives thereof. A nucleotide is a phosphate group attached to 5'-position of sugar, and a base bound to the sugar at 1'-position. The sugar is deoxyribose. The bases may be adenine, guanine, thymine and cytosine, and derivatives thereof. An aptamer sequence may include aromatic groups that are not the indicated bases.

The term "nucleotide" used herein refers to nucleotides, which may be natural nucleotides (for example, ATP, TTP, GTP, CTP, UTP) or modified nucleotides. The modified nucleotides are nucleotides comprising bases, such as, for example, adenine, guanine, cytosine, thymine and uracil, xanthine, inosine, and queuosine, which have been modified by submitting or adding one or more atoms or groups. Some examples of types of modifications that can comprise nucleotides, which base moieties are modified, includes, but not limited to, alkylated, halogenated, thiolated, aminated, amidated, or acetylated bases in various combinations. More specific examples include 5-propinyluridin, 5-propynyluridine, 5-propynylcytidine, 6-methyladenine, 6-methylguanine, N5N-dimethyladenine, 2-propyladenine, 2-propylguanine, 2-aminoadenine, 1-methylinosine, 3-methyluridine, 5-methylcytidine, 5-methyluridine and other nucleotides having a modification at the 5 position, 5-(2-amino)propyl uridine, 5-halocytidine, 5-halouridine, 4-acetylcytidine, 1-methyladenosine, 2-methyladenosine, 3-methylcytidine, 6-methyluridine, 2-methylguanosine, 7-methylguanosine, 2,2-dimethylguanosine, 5-methylaminoethyluridine, 5-methyloxyuridine, deazanucleotides such as 7-deaza-adenosine, 6-azouridine, 6-azocytidine, 6-azothymidine, 5-methyl-2-thiouridine, other thio bases such as 2-thiouridine and 4-thiouridine and 2-thiocytidine, dihydrouridine, pseudouridine, queuosine, archaeosine, naphthyl and substituted naphthyl groups, any O- and N-alkylated purines and pyrimidines such as N6-methyladenosine, 5-methylcarbonylmethyluridine, uridine 5-oxyacetic acid, pyridine-4-one, pyridine-2-one, phenyl and modified phenyl groups such as aminophenol or 2,4,6-trimethoxy benzene, modified cytosines that act as "G-clamp" nucleotides, 8-substituted adenines and guanines, 5-substituted uracils and thymines, azapyrimidines, carboxyhydroxyalkyl nucleotides, carboxyalkylaminoalkyl nucleotides, and alkylcarbonylalkylated nucleotides. Modified nucleotides also include those nucleotides with modified their sugar moiety (for example, 2'-fluoro or 2'-O-methyl nucleotides), as well as nucleotides having sugars or analogs thereof that are not ribosyl. For example, the sugar moieties may be or may be based on mannoses, arabinoses, glucopyranoses, galactopyranoses, 4'-thioribose, and other sugars, heterocycles, or carbocycles. The term "nucleotide" also means, as known in the art, a universal base. By way of example, universal bases include, but are not limited to, 3-nitropyrrole, 5-nitroindole, or nebularine. Modified nucleotides include labeled nucleotides such as radioactively, enzymatically, or chromogenically labeled nucleotides.

After identification of an aptamer sequence, the aptamer can be synthesized by any method known in the art. In one embodiment, aptamers can be produced by chemical synthesis of oligonucleotides and/or ligation of short oligonucleotides.

In one aspect of the disclosure, aptamers are modified so that they have an increased circulation time after administration to an individual. In one embodiment, the aptamer nucleotides are joined together by phosphate bonds. In another embodiment, one or more internucleotide bonds are modified bonds, for example, bonds resistant to nucleases. The term "modified internucleotide bond" covers all modified internucleotide bonds that are known in the art or will be known from the description and that a person skilled in the art considers it possible to use in the context of the present disclosure. Internucleotide bonds can be formed between counterions, wherein this term refers to those counterions and any coordination complexes that can be formed between internucleotide bonds. Modifications of internucleotide bonds include, but are not limited to, phosphorothioates, phosphorodithioates, methylphosphonates, 5'-alkylenphosphonates, 5'-methylphosphonate, 3'-alkylenphosphonates, boron trifluoride etherate, boron esters of phosphoric acid and selenophosphates of the 3'-5' bond or 2'-5' bond, phosphotriesters, thionoalkylalkyl-phosphotriester, hydrophosphoric bonds, alkylphosphonates, alkylphosphonothioates, arylphosphonothioates, phosphoroselenoate, phosphorodiselenoates, phosphinates, phosphoramidates, 3'-alkylphosphoramidates, aminoalkylphosphoramidates, tionophosphoramidates, phosphoropiperazidates, phosphoronyl thioates, phosphoroanilidates, ketones, sulfones, sulfonamides, carbonates, carbamates, methylenhydrazo, methylendimethylhydrazo, formacetals, thioformacetals, oximes, methylenimines, methylenmethylimines, thioamidates, bonds to riboacetyl groups, glycine aminoester, silyl or siloxane bonds, alkyl or cycloalkyl bonds with or without heteroatoms, for example from 1 to 10 carbons, which can be substituted or nonsubstituted, and/or substituted and/or include heteroatoms, bonds with morpholino structures, amides, polyamides, wherein the bases can be either directly or indirectly attached to an aza-nitrogen atom in the backbone, and a combination of such modified internucleotide bonds. In another embodiment, the aptamers include 5'- or 3'-terminal blocking groups to prevent degradation from nucleases (for example, inverted deoxythymidine or hexylamine).

In another embodiment, the aptamers are bound to conjugates that increase their circulation time, for example, that reduce nuclease degradation or renal filtration of the aptamers. Conjugates can include, for example, amino acids, peptides, polypeptides, proteins, antibodies, antigens, toxins, hormones, lipids, nucleotides, nucleosides, sugars, carbohydrates, polymers such as polyethylene glycol and polypropylene glycol, and analogues or derivatives of all of these types of compounds. Additional examples of conjugates also include steroids such as cholesterol, phospholipids, di- and triacylglycerols, fatty acids, hydrocarbons that optionally comprise substituents, enzymes, biotin, digoxigenin, and polysaccharides. Other examples include thioethers such as hexyl-S-trityl thiol, thiocholesterol, acyl chains, such as dodecanediol or undecyl groups, phospholipids, such as di-hexadecyl-rac-glycerol, triethylammonium 1,2-di-O-hexadecyl-rac-glycero-3-H-phosphonate, polyamines, polyethylene glycol, adamantane acetic acid, palmityl groups, octadecylamine groups, hexylaminocarbonyloxycholesterol, farnesyl, geranyl and geranylgeranyl groups, for example, polyethylene glycol, cholesterol, lipids or fatty acids. Conjugates also may comprise a label. For example, conjugates may be fluorophores. Conjugates may include fluorophores such as TAMRA, BODIPY, cyanine derivatives, such as Cγ3 or Cγ5 Dabsyl, or other suitable fluorophores known in the art. A conjugate may be attached at any end of a nucleotide, if it is possible, and it does not affect a desired aptamer activity, for example, at the 3' or 5' ribosyl end. A conjugate essentially provides a desired activity, i.e. the aptamer binds thrombin at least with the dissociation constant of less than 1 µM.

"Thrombin time" refers to clotting time in the presence of exogenous mammalian, including human, thrombin.

The numbering of all bases in an aptamer sequence is indicated in the 5'-3' direction. Thus, "G1" means guanine at position 1 in a DNA sequence.

The term "inner loop" refers to unpaired (not forming Watson-Crick interactions) nucleotides on both strands of a nucleotide sequence, which are opposite to each other in the secondary structure.

"Force fields" are a combination of parameters for computer simulation of molecular mechanics and dynamics.

All analytical techniques and methods used in the present disclosure are well known to a person of ordinary skill in the art.

Thus, the term "computer molecular dynamics simulation" refers to a method wherein the time evolution of a system of interacting atoms or particles is followed by integrating their equations of motion. For describing the motion of atoms or particles there is used classical mechanics. The law of particle motion is determined according to analytical mechanics. Interatomic forces are in the form of the classical potential forces (as the gradient of the potential energy of a system).

"Force fields" are a combination of parameters for computer simulation of molecular mechanics and dynamics.

The term "X-ray analysis" refers to the X-ray diffraction analysis that is one of the diffraction methods for studying the structure of a substance. This method is based on the phenomenon of X-ray diffraction on a three-dimensional crystal lattice. The structural models of a substance obtained by this method are called XSA models.

The term "static model of a structure" refers to the tertiary polymer structure that does not contain information about the evolution of the structure with time.

The term "path length" refers to the number of iterations for integrating the equations of the system motion. This term defines a period of time of observing the evolution of a system in computer molecular dynamics simulation.

The term "therapeutically effective amount" refers to such an amount of a therapeutic agent that is sufficient to inhibit thrombin and/or eliminate the symptoms of thrombosis.

The term "prevention" means reducing the risk of clotting.

The term "treatment" refers to any type of the treatment that includes: improvement of patient's condition, complete or partial recovery of a patient, slowing the development of a disease and the like.

The term "pharmaceutically acceptable" refers to such an amount of compounds or compositions that is suitable for administration to an individual for the treatment or prevention, wherein such amount does not cause side effects in view of the severity of a disease and the need for treatment.

The term "specifically bind" as used herein refers to a molecule (for example, a DNA aptamer) that binds to a target (for example, thrombin) with at least five-fold higher efficiency than any molecule that is not a target, for example, at least with 10-, 20-, 50- or 100-fold higher efficiency.
In one embodiment, the present disclosure relates to pharmaceutical compositions comprising the DNA aptamers according to the present disclosure and a pharmaceutically acceptable carrier. In one embodiment, the pharmaceutical composition comprises a therapeutically effective amount of the DNA aptamers according to the present disclosure.
In another aspect, the present disclosure relates to methods of using the DNA aptamers according to the present disclosure to inhibit thrombin. Such methods can be carried out both in vitro and in vivo. The methods can be used to treat diseases and conditions associated with an undesirable thrombin activity, in particular, to treat thrombosis. In addition, the DNA aptamers according to the present disclosure can be used in diagnostic methods. Inhibition of thrombin achieved by the DNA aptamer according to the present invention can be assessed by any method known in the art.

In one embodiment, the present invention relates to methods for treating or preventing diseases or conditions when thrombin activity is undesirable, in particular for preventing thrombosis in a mammal, including a human, wherein the method comprises administering the DNA aptamers according to the present invention to an individual with such disease or condition. The term "disease or condition associated with an undesirable thrombin activity" refers to any disease or condition for which inhibition of thrombin could have a therapeutic or prophylactic effect. Non-limiting examples of diseases and conditions associated with an undesirable thrombin activity are thrombophlebitis, thrombosis in the arterial system, thrombosis in the coronary circulation, myocardial infarction, cerebral thrombosis, cerebral stroke, disseminated intravascular coagulation syndrome, and the like.

Since the inhibition of thrombin is a necessary step in the treatment or prevention of diseases or conditions associated with an undesirable thrombin activity, in one embodiment, the present disclosure relates to a method of inhibiting thrombin by the anti-thrombosis aptamer according to the present disclosure. In one embodiment, the present disclosure relates to a method of inhibiting thrombin in vitro. In another embodiment, the present disclosure relates to a method of inhibiting thrombin in vivo, in particular in a mammalian body, in the preferred embodiment - in a human body.

The DNA aptamers according to the present invention can be administered together with other compounds (for example, therapeutic agents) or in parallel with another treatment (for example, surgery) for the treatment and prevention of the indicated diseases or conditions. Other compounds can be administered simultaneously with the DNA aptamers according to the present invention, in parallel with the DNA aptamers according to the present invention, or other compounds and the DNA aptamers can be administered at different time intervals. In particular, a DNA aptamer and another compound may be administered as a part of a single composition.

In one embodiment, the present disclosure relates to the use of the DNA aptamers according to the present disclosure for diagnostic purposes. The DNA aptamers according to the present disclosure can be used as binding agents in the assessment of thrombin levels. Such assessment is useful for diagnosing a disease or condition associated with a thrombin level. In addition, the DNA aptamers according to the present disclosure may be used as an imaging agent in vivo or in histological analyses. Analyses and methods suitable for these purposes are well known to those skilled in the art. Diagnostic analysis can be made in vitro, for example in patient-derived samples (for example, tissue samples), or in vivo. The DNA aptamers according to the present disclosure may be labeled by methods and with labels well known in the art.

The present invention first of all relates to the treatment of diseases and conditions associated with an undesirable thrombin activity in a mammal, including human. Furthermore, the present disclosure relates to the diagnosis of diseases and conditions associated with an undesirable thrombin activity in a mammal, including human.

The DNA aptamers according to the present invention can be prepared in the form of compositions to be administered together with a pharmaceutical carrier according to known methods (for example, Remington, The Science And Practice of Pharmacy (9th Ed. 1995)). The preparation of the pharmaceutical composition according to the present disclosure comprises mixing the DNA aptamer with a suitable carrier. The carrier is required to be inactive, i.e. it does not interact with the DNA aptamer according to the present disclosure and does not have negative reactions on a mammal body, in particular a human. The carrier may be liquid and/or solid and can be introduced into a composition comprising the DNA aptamer according to the present disclosure to obtain a standard dosage form.

The composition according to the present disclosure can be administered by any method known to a person skilled in the art.

A therapeutically effective dose of the DNA aptamer according to the present disclosure in the composition, as well known to those skilled in the art, depends on factors such as age, general patient condition, and route of administration. The determination of a particular dose for each case is a routine procedure for the pharmacist. As a rule, a standard dosage form comprising from about 0.001 or about 0.01 to about 250 or 500 mg/kg is therapeutically effective.

### Detailed description of preferred embodiments

The following examples illustrate preferred materials and methods according to the present invention.

### Comparitive Example 1

Computer molecular dynamics (MD) simulation makes it possible to study in detail the factors affecting the stability of the structure or fluctuation of atoms in a biopolymer structure.

Today, two variants of the static structures of the aptamer 15TVA are known, they are: data of nuclear magnetic resonance (NMR) and data of x-ray structural analysis (XSA). As a result of MD simulation, the NMR model of 15TVA has not significantly changed. The PCA model of 15TVA, on the contrary, has changed its initial conformation - the spatial structure of G-quadruplex has acquired a conical geometry that is unusual for G-quartet structures.

Although, the loops of G-quadruplex structures are very difficult to be MD simulated by using the current computer force fields, in the NMR model, the central TGT loop and the lateral TT loops of the finite dynamic structure differs very little from the structure obtained by NMR spectroscopy. This positive result makes it possible to construct dynamically stable models of various 15TGT structures by MD methods (molecular dynamics simulation).

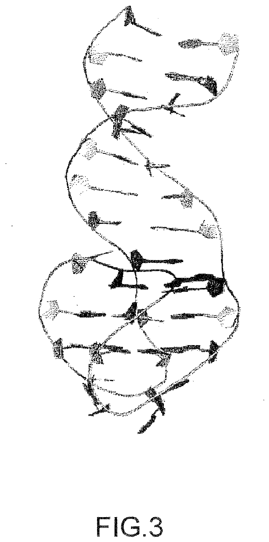

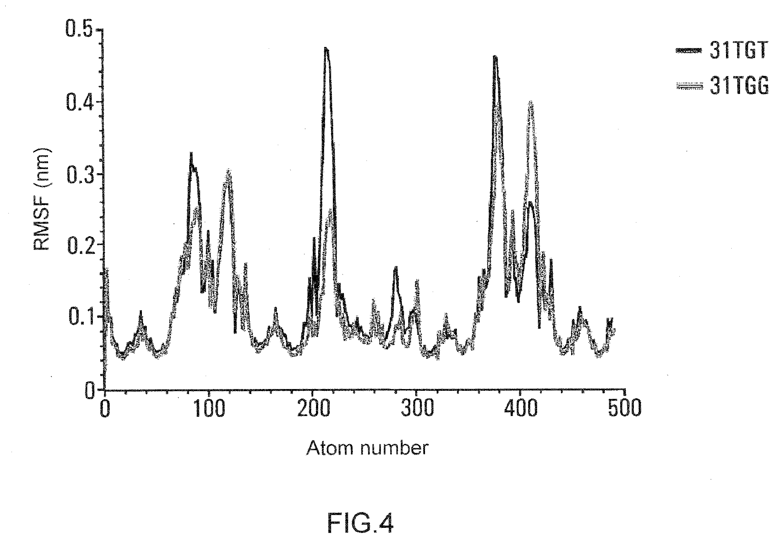

The aptamer 31TGT inhibited thrombin better than 15TGT; in addition, the structure thereof had additional nucleotides at both ends of the molecule. The following model of the spatial structure of the aptamer 31TGT was constructed by computer simulation methods: the ends of the molecule locked into a duplex section and connected to the quadruplex by internal loops, two nucleotides per each (2x2). To obtain a static model, MD simulation was performed. The inside loops 2x2 can form additional interactions with the TGT loop by means of three nucleotides that are above the upper quartet. It should be noted that nucleotide T9 of the lateral TGT loop is not involved in the formation of that specific structure of stacking interactions. To stabilize the structure by forming strong stacking interactions according to the present disclosure, this nucleotide T was substituted to a nucleotide G. The guanine residue forms more stable stacking interactions and has a great potential to form hydrogen bonds. The obtained model of the new aptamer variant was subjected to MD. The monitoring time (the length of the trajectory) was 1 microsecond, or 500 million iterations. Within the monitoring time period, the model of the spatial structure of the new aptamer variant demonstrated an increased stability in MD. Fluctuations of the guanine residues belonging to the quadruplex were significantly lower (Figure 4). The appearance of the second guanine in the lateral TGT loop allowed additional interactions (Fig.3). All interactions were stable and did not change after the molecule reached an equilibrium state and up to the end of the trajectory. The loop site formed a structure wherein all of elements were involved into interactions. The G-quartets were stable and were in parallel planes. The duplex site also demonstrated an increased stability.

The efficiency of an inhibitory effect of the new aptamer variant on thrombin was determined, wherein the structure of the new aptamer was designed by the method of obtaining stable aptamer according to the present disclosure. For this purpose the aptamer variant was chemically synthesized and the inhibitory capability thereof was determined in the hydrolysis of fibrinogen by thrombin.

The modified aptamer with the sequence NₓGTAGGTTGGTGGGGNTGGRRCNₓ was designated by index DTI-P91.

Similarly, aptamers DTI-G84, DTI-33 (the aptamer according to the invention), DTI-r91, DTI-A6 and DTI-Fx7, which nucleotide sequences were disclosed above, were obtained by using MD simulation.

### Example 2

The DNA aptamers according to the invention (and according to the present disclosure) were synthesized according to a standard procedure of solid-phase synthesis (Oligonucleotide Synthesis, MJ Gait ed., 1984; Sambrook, Fntsch & Maniatis, Molecular Cloning: A Laboratory Manual, Second Edition (1989).

Aptamers were obtained by a method of automated solid-phase synthesis on the MerMade 48 synthesizer by using β-cyanoethyl-N,N-bis-diisopropylamidophosphites of nucleosides as reagents. A SiO₂-based polymer (CPG - Controlled Pore Glass) modified by aminopropyl groups was used as a carrier. Columns with the carrier comprised the first nucleotide of the aptamer that was attached to an aminopropyl group on the carrier surface by a succinyl linker connecting the amino group and 3'-hydroxyl group of the nucleotide.

The synthesis consisted of a few basic steps:
1) Detritylation - removing a protecting dimethoxytrityl group from 5'-end of the elongated chain by trichloroacetic acid.
2) Attachment. Phosphoramidite of an attached nucleoside was activated by a catalyst - ethylthiotetrazole. This mixture was fed into a column, wherein it contacted with an oligonucleotide precursor. The 5'-hydroxyl group of the precursor released from the dimethoxytrityl protection reacted with phosphoramidite, forming a phosphite ester bond.
3) Capping. After completion of the previous step, there was an amount (0.1-1%) of unreacted 5'-OH groups that were deactivated by acetic anhydride, preventing thereby the further chain elongation on these groups and the formation of oligonucleotides that were shorter than the target one.
4) Oxidation. The phosphite ester linker was converted into a phosphate linker by oxidation with an iodine solution in pyridine.

When the synthesis was completed, the oligonucleotide was removed from the carrier with ammonia.

### Example 3

To confirm the inhibition of thrombin by the aptamers according to the invention (and according to the present disclosure) and to study their anticoagulant activity, the time of inhibition of thrombin (in seconds) was assessed at a temperature of 270°C in the system described in Table 1.

| Human plasma | 2 µl |
|---|---|
| Human thrombin | 13 IU |
| NaCl | 70 mM |
| KCl | 2.5 mM |
| MgCl₂ | 0.5 mM |
| CaCl₂ | 0.5 mM |
| Tris-Ac | 10 mM |
| pH | 7.4 |
| Aptamer | Various concentrations |

The values of inhibition of thrombin were obtained in seconds by a spectrophotometric method for assaying coagulation of human plasma in the presence of various concentrations of the aptamer, from 0 to 60 nM (see Figure 1).

Since the computer simulation provides real variants of modified structures, but does not assess the efficiency of self-assembling a polymer in a solution, the authors of the invention have studied the efficiency of the claimed sequences as anticoagulant agents.

### The first parameter: prothrombin time

Prothrombin time is the time required for formation of a fibrin clot after adding a calcium chloride solution and standardized tissue thromboplastin. Prothrombin time characterizes the activity of the so-called prothrombin complex (factors V, VII, X and prothrombin per se - factor II). The result of the study was expressed in seconds (prothrombin time). The prothrombin test is a screening test simulating the external pathway of blood coagulation, thus, the test characterizes the first (protrombin formation) and the second (trombin formation) phases of plasma homeostasis and reflects the activity of the prothrombin complex. The test is used to assess the deficiency of the prothrombin complex coagulation factors and the external coagulation pathway. The results of the test, in case of the normal amount and quality of fibrinogen, depend on the amount of factors VII, V, X, II, and the activity thereof. The main factors of the prothrombin complex are formed in the liver, for this reason this test is often used to assess its activity in the protein biosynthesis.

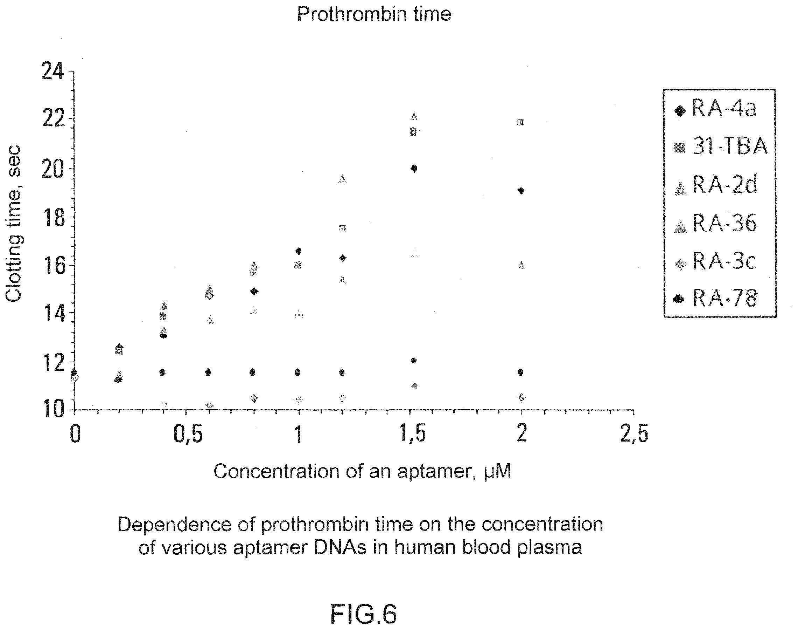

The international normalizing ratio (INR) is a more standardized test. In the most cases, during the treatment by indirect-action anticoagulant drugs (anticoagulants), it is sufficient to achieve an increase in INR within a range of 2 to 3, which corresponds to an increase in prothrombin time by 1.3-1.5 times compared with a baseline. Figure 6 shows the dependence of prothrombin time on the concentration of various aptamer DNAs in human blood plasma.

### The second option: thrombin time

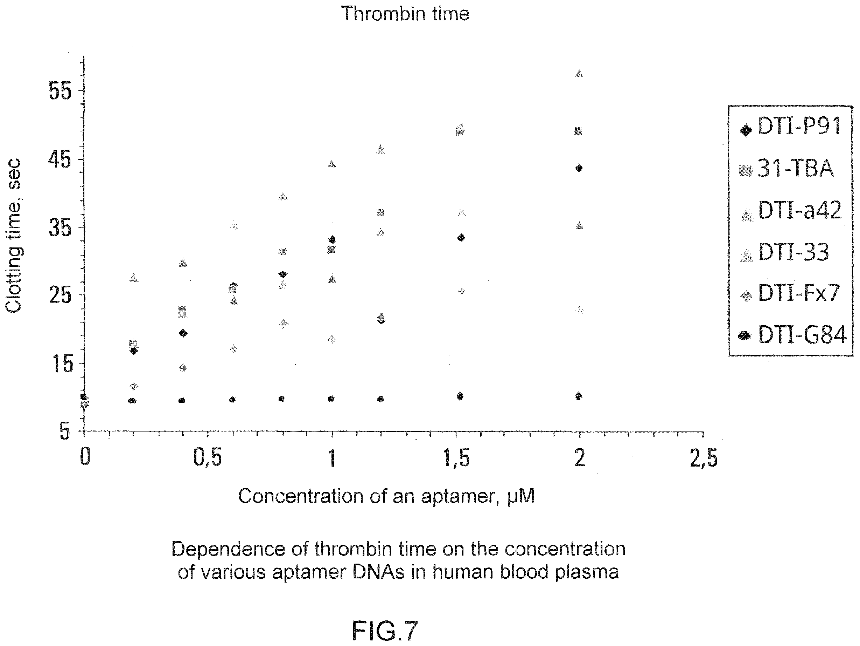

Thrombin test shows the time required for formation of a fibrin clot after adding thrombin. This test characterizes the kinetics of the final step of blood coagulation - the rate of conversion of fibrinogen to fibrin. Thrombin time depends only on the concentration of fibrinogen and the activity of thrombin inhibitors (antithrombin III, heparin, paraproteins). This test is used for the assessment of the third phase of blood coagulation - the formation of fibrin and the condition of natural and pathological anticoagulants. The determination of thrombin time is one of the most common methods of monitoring heparin and fibrinolytic therapy, and is also used for diagnosing hyperfibrinolytic states, afibrinogenemia and dysfibrinogenemia. Complete non-coagulability is observed immediately after intravenous administration of a large dose of heparin and in the terminal phase of acute DIC-syndrome. Figure 7 shows the dependence of thrombin time on the concentration of various aptamer DNAs in human blood plasma.

### Discussion of the results

It has been found that at lower concentrations, the new aptamer variant DTI-P91 is more effective thrombin inhibitor in hydrolysis of fibrinogen than the known aptamer 31 TGT (Figure 1).

### Conclusion

In support that stacking interactions of the heterocyclic bases of the TGT loop with the upper G-quartet make a significant contribution to the overall structure of the anti-thrombosis aptamer G-quadruplex, the properties of the aptamers comprising the substitution of a residue G to a residue C in the TGT loop were compared. The obtained data clearly demonstrate the importance of stacking interactions. The direct substitution of the central purine G in the TGT loop to smaller heterocycle, pyrimidine C, by definition, dramatically reduces stacking interactions, leading to a significant decrease in inhibition. And vise versa, the substitution of pyrimidine T adjacent to the central base in the TGT loop to larger purine G leads to an increase in inhibitory activity of the aptamer - an increased thrombin time. A similar effect of stacking interactions may be also modulated by derivatives of heterocyclic bases and by similar aromatic systems.

The importance of the described stacking interactions with the upper G-quartet in stabilization of the overall G-quadruplex structure is indirectly confirmed by another fact. As was earlier noted, the traditional factor for the stability of a quadruplex is a K⁺ ion that is coordinated by oxygen atoms in the center of the quadruplex, and thereby stabilizes the overall structure. The coordination of the ion has a dynamic character, i.e. the ion is mobile and can communicate with the external environment. The presence of additional stacking interactions blocks the release of the ion from the quadruplex into a solution through the upper quartet. A barium ion, Ba2⁺, behaves differently than a K⁺ ion, its own co-ordination is more effective and it is stable in the quadruplex structure. Moreover, additional stacking interactions above the quartet do not have a significant effect on the stabilization of the overall structure.

### Description of a method for determining thrombin activity and an inhibitory effect of the aptamer on the hydrolysis of fibrinogen by thrombin

Biochemical methods for determining thrombin activity can be divided into two groups. In the first group low-molecular-weight chromogenic substrate analogues, which are digested by the active center of enzyme to form a colored product, are used. Examples of such substrates are H-D-Phe-Pro-Arg-p-nitroanilide (FPR), H-D-Phe-Pro-Phe-p-nitroanilide (FPF) and longer peptides, for example, H-Gly-D-Phe-Pip-Arg-Ser-(Gly)₄-Lys-Cys-NH₂ (Pip-pipecolic acid, Cys-NH₂ - Chromophore). The obtained data characterize only the activity of the thrombin catalytic center, and it is more correct to use the methods of the second group.

The second group includes methods that consider not only activity of the catalytic triad, but also binding of natural substrates to the absorption site of protease. Enzymatic thrombin activity may be defined from curves of the accumulation of one of the digested products of such substrates as PAR1, PAR4, protein C (Protein C) or fibrinogen. The use of HPLC allows quantification of PAR1-peptide (41 amino acid residues), PAR4- peptide (47 amino acid residues), protein C-peptide (41 amino acid residues), and fibrinopeptides A and B (16 and 14 amino acid residues, respectively).

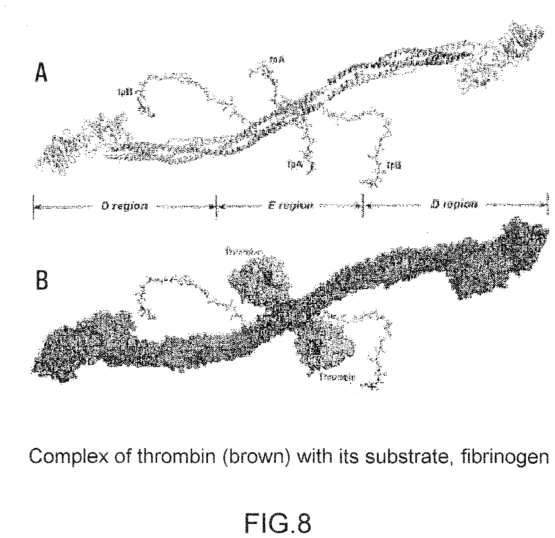

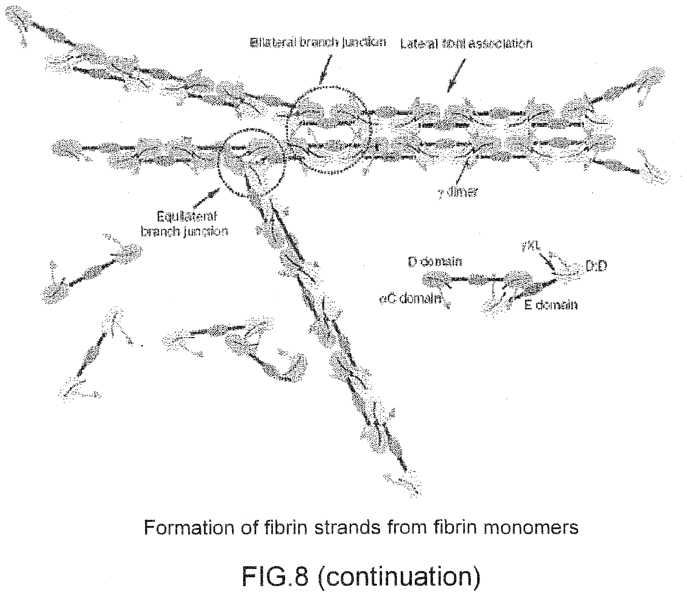

However, specialized expensive HPLC equipment in general is not available for routine use in private laboratories, which is the reason for the development of methods based on the detection of thrombin-cleavable proteins, rather than detached peptides. In this field, methods only for determining fibrin - a product of proteolysis of fibrinogen by thrombin, have been developed. Fibrin spontaneously and reversibly aggregates by forming fibrin strands. Despite the fact that methods of the quantification of monomer fibrin (so-called fibrin-monomer) on the basis of immunoenzyme analysis ELISA have been developed for clinical applications, in laboratory studies it is more convenient to determine the level of fibrinogen conversion according to the formation of fibrin strands (Fig. 8).

The essence of the optomechanical method consists in stirring a reaction mixture with a stir bar, which leads to winding, on the stir bar, fibrin strands formed in the reaction and slowing down the rotation of the stir bar up to its full stop. The stop time of the stir bar is recorded as clotting time. Despite the fact that the obtained value is to some extent conventional and there is no any correlation with kinetic parameters, standardization of reagents and reaction conditions makes the clotting time to be a well-reproducible parameter. The optomechanical method is mostly used only in clinical laboratories due to a small range of determined thrombin activity (0.3-0.6 IU per 300 ml of a sample), a significant result error (50%), and the need for highly specialized equipment - instruments such as Fibrintimer.

The results of the opticalmechanical method do not reliably reflect the aggregation of fibrin in case of some genetic mutations in a series of diseases; therefore, to study aggregated fibrin it is more preferable to use spectrophotometric methods.

Spectrophotometric methods for determining the thrombin activity do not use the step of stirring a solution during the reaction. At the initial stages of proteolysis, a solution of aggregating fibrin behaves like a colloidal solution that, in the course of the reaction, transforms to gel formed by the three-dimensional framework of the aggregated fibrin and a solution of low-molecular-weight compounds adsorbed by this gel. The reaction is accompanied by an increase in the turbidity and viscosity, and, therefore, nephelometry, turbidimetry and viscometry, respectively, can be used for monitoring thereof. Currently, turbidimetry-based methods, which an undoubted advantage is the use of spectrophotometers (compact and available devices for laboratory applications) for detection, are most developed. The light scattering by a sample of aggregating fibrin is detected by a spectrophotometer as radiation absorption. The resultant value is a complex function of the shape, size and concentration of the aggregated fibrin particles; however, the obtained absorption/reaction time dependences allow one to define a number of parameters with established dependency on the enzyme and fibrinogen concentration and temperature.

In general, the wavelength of incident light is selected to be of 330 to 660 nm, i.e. within the range of the transparency of colorless proteins. So called "lag" time, T_{lag}, is selected as a parameter that has linear dependency on the thrombin concentration in a range of 0.03-0.3 IU/sample (error of about 10%), i.e. the time period during which there is no absorption of a sample. Parameter that is proportional to the fibrinogen concentration is the height of the saturation plateau.

Thus, the development of the turbidimetric method for determining the thrombin activity and, therefore, the effective and economical method for determining a thrombin inhibitor activity, seems to be perspective. This method has been developed and used to determine an inhibitory effect of the aptamers and the type of inhibition of fibrinogen.

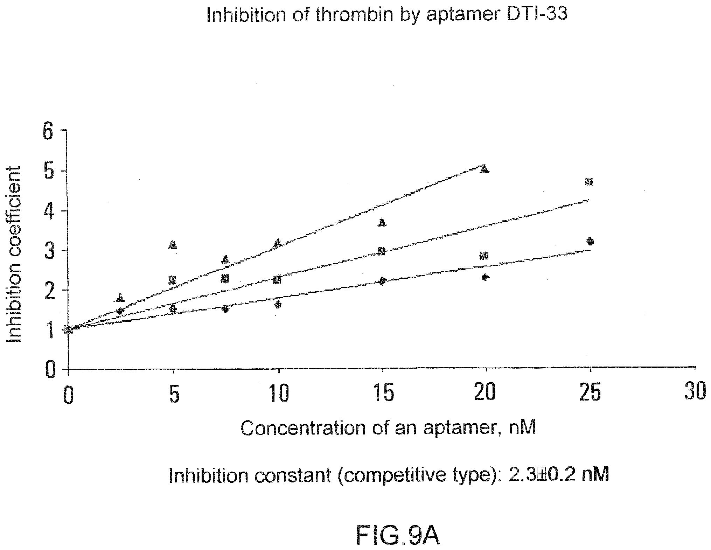

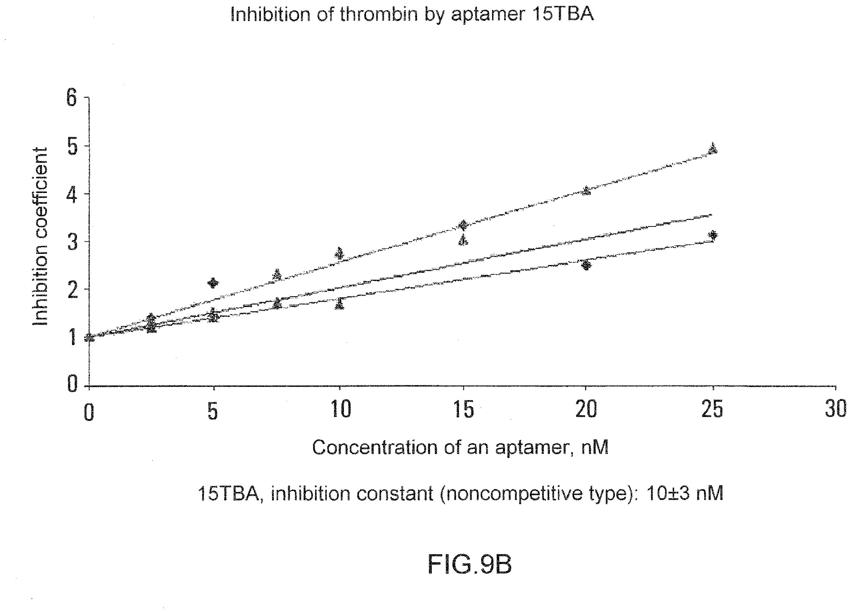

Conditions of the experiment:
Thrombin - 4 nM, fibrinogen - 2.2 µM (blue), 1.1 µM (green), 0.56 µM (red), 27°C.
The results are shown in Figures 9A and 9B.

The designed aptamer, unlike 15TVA, has improved the inhibition constant, and competes with fibrinogen for binding to thrombin.

### Preliminary determination of aptamer toxicity in animals

### Protocol for preparing solutions:

5 tubes with 100 ml of a saline solution;
5 tubes with 100 ml of a 10 mM KCl solution;
5 tubes with 100 ml of a 10 mM KCl solution comprising 81 mg/ml of aptamer DTI-33.

All solutions were hold for 5 minutes at 95°C and left to cool at room temperature, and were encrypted.

15 rats were used in the study, the animals showed various deviations from the normal behavior; however, there were no differences between groups, there were no fatal cases, among the rats it was impossible to distinguish animals comprising aptamers.

The aptamer DTI-33 has no toxicity to rats.

### Comparative study of aptamer affinity to thrombin by capillary electrophoresis

The binding of 5 aptamers with a human α-thrombin was performed under the same conditions by a CGE (capillary gel electrophoresis) method. The concentration of aptamers was 250 nM. This concentration was selected on the basis of the detection limit for an aptamer with the lowest fluorescence signal intensity. It is assumed that the selected concentration is higher than the value of K_{D} (apparent dissociation constant) and, therefore, it is impossible to estimate K_{D} values directly from the binding curves; however, the qualitative assessment of the aptamer affinity to thrombin is possible according to the slope of the curves and Bmax.

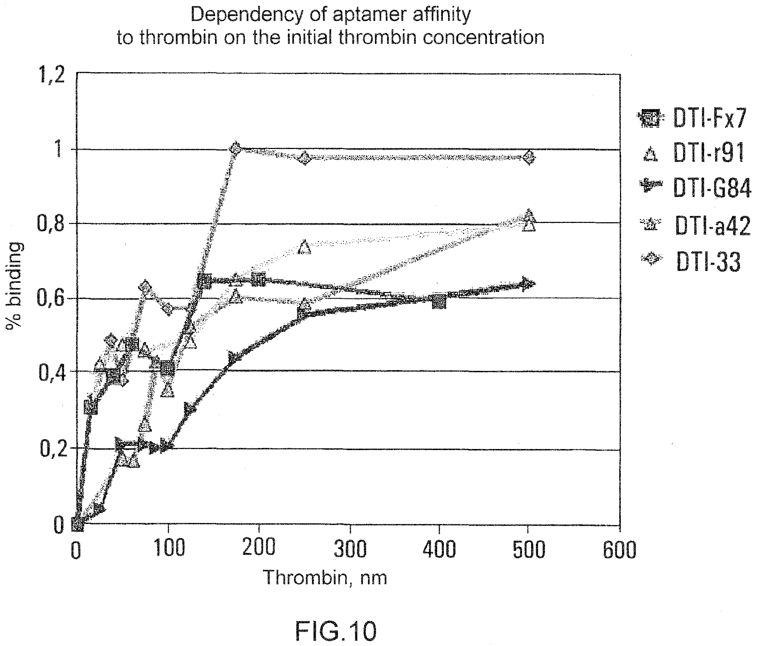

Among the selected aptamer samples, DTI-33 possesses the maximal affinity. All aptamers demonstrate high-affinity binding to thrombin, except DTI-G84 (Fig.10).

### Procedure of the experiment for administering aptamers to rats

Several schemes for administering aptamers to laboratory animals have been tested. In December 2009, a mouse was chosen as an animal model. A technique of administering aptamers to the sublingual vein in a volume of 100 ml was developed. However, a blood volume required for follow-up analysis (200 ml) could be taken only one time from the heart cavities. In connection with the above disadvantage, it was decided to change the animal model and, finally, the rat was chosen as an experimental model.

Most of the experiments were conducted on female Wistar rats weighing about 200 g.

### Scheme of the experiment

1. An animal was weighed on an electronic balance, and a 20% chloral hydrate solution (on the basis of 1000 ml of the solution per 200 g of animal body weight) was administered intraperitoneally.
2. After achieving deep narcotic sleep, incisions were made in the area of the jugular vein.
3. The jugular veins on both sides were exposed, and an aptamer solution in an amount of 100 ml was injected into one of both under visual control.
4. After certain periods of time (1 min after the administration of aptamer, 3 min, 5 min, 7 min, 10 min, 15 min and 20 min) from the symmetrical vein, the blood of the animal was taken in amount of 200 ml into a syringe comprising 20 ml of a 3.8% sodium citrate solution.
5. The blood was transferred from the syringe to a microcentrifuge tube, intensively shaken to achieve uniform mixing of sodium citrate and sent for further analysis.

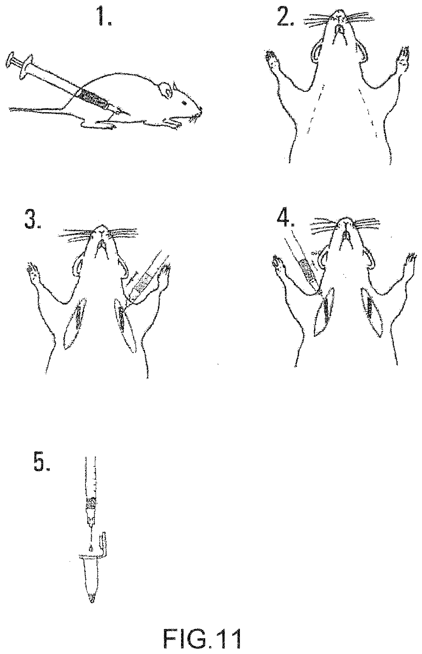

The scheme of the experiment is shown in FIG.11. The numerals on the figure correspond to the serial number of the steps according to the text.

### Studies of the inhibiting capacity and stability of aptamers in rats

Preparations of aptamers were preformed as follows:
1) an aptamer was diluted with a saline solution to a concentration of 1 to 5 mM;
2) 3 M potassium chloride was added to the aptamer solution up to a final concentration of 10 mM;
3) the preparation was heated at 95°C for 5 min; and
4) left to cool at room temperature.

Before the administration to a rat, a required amount of an aptamer was diluted with a saline solution to a volume of 100 µl. The administration to a rat was performed as previously described.

Blood samples taken at different time intervals was mixed with a 3.8% sodium citrate solution in a ratio of 9 to 1, and then centrifuged at 1500 rpm for 10 min. The resultant animal citrated plasma was analyzed with an optical coagulometer in two tests: thrombin and prothrombin times. The control values of rat blood plasma were obtained by averaging the values of five animals.

It has been found that aptamers does not have any effect on prothrombin time of rat plasma, which reflects the lack of interactions between the aptamers and rat thrombin (therefore, a high specificity for human thrombin, see the data relating to affinity and inhibition). Thus, prothrombin time is a reliable indicator of the general state of homeostasis in rats, both in the presence of an aptamer and without it.

Thrombin time, as expected, was sensitive to the concentration of an injected aptamer. Since the effect was measured by the exogenous human thrombin, exactly this parameter should be considered as an assessment criterion for removal of aptamers from a rat body. However, it should be noted that the sensitivity of the test depends on the nature of an aptamer (that was studied in vitro in human plasma).

Figures 12 A-D show the average dependences of "thrombin time - time after administration" for six aptamers. The concentrations of aptamers are presented in micrograms per 1 g of animal body weight.

The average time of the presence of an aptamer in animal blood was 10 min. The high removal rate is an advantage because it allows precise control of the state of homeostasis within a selected time range.

Chemical modifications of aptamers do not lead to the loss of their inhibitory activity

The aptamer DTI-33 with a polyethylene glycol (PEG) group at the 3'-end of a nucleotide was synthesized to determin in principle the possibility of designing aptamers with a longer time of action. It is known that the addition of PEG groups can significantly increase the time of the presence of target molecules in blood.

The addition of a PEG group changes insignificantly the inhibiting properties of the aptamer DTI-33 (Fig.13).

## Claims

1. A two-quadruplex DNA aptamer for use in a method of preventing or treating a condition associated with an undesirable thrombin activity, wherein the aptamer is an oligodeoxyribonucleotide with a nucleotide sequence of the general formula of:
GGTTGGTGTGGNTGG-Tx-GGTTGGTGTGGNTGG;
where Tx is one or more bases T.

2. The aptamer for the use according to claim 1, wherein the condition associated with an undesirable thrombin activity is thrombosis.

3. The aptamer for the use according to claim 1, wherein the condition associated with an undesirable thrombin activity is thrombophlebitis, thrombosis in the arterial system, thrombosis in the coronary circulation, myocardial infarction, cerebral thrombosis, cerebral stroke, disseminated intravascular coagulation syndrome.

4. The aptamer for the use according to any of claims 1-3, wherein the aptamer is to be administered together with other compounds or in parallel with another treatment.

5. The aptamer for the use according to claim 4, wherein the other treatment is surgery.

6. The aptamer for the use according to claim 4, wherein the other compounds can be administered simultaneously with the administration of the aptamer, in parallel with the administration of the aptamer, or wherein the compounds and the aptamer can administered at different time intervals.

7. The aptamer for the use according to claim 4, wherein the aptamer and the other compounds can be administered as a part of a single composition.

8. A two-quadruplex DNA aptamer for use in preventing and treating a condition associated with an undesirable thrombin activity, wherein the aptamer is an oligodeoxyribonucleotide with a nucleotide sequence of the general formula of:
GGTTGGTGTGGNTGG-Tx-GGTTGGTGTGGNTGG;
where Tx is one or more bases T.

## Patentansprüche

1. Zwei-Quadruplex-DNA-Aptamer zur Verwendung in einem Verfahren zum Vorbeugen oder Behandeln eines Zustands, der mit einer unerwünschten Thrombinaktivität assoziiert ist, wobei das Aptamer ein Oligodesoxyribonukleotid ist, mit einer Nukleotidsequenz der allgemeinen Formel:
GGTTGGTGTGGNTGG-Tx-GGTTGGTGTGGNTGG;
worin Tx eine oder mehr T-Basen ist.

2. Aptamer zur Verwendung gemäß Anspruch 1, wobei der Zustand, der mit einer unerwünschten Thrombinaktivität assoziiert ist, Thrombose ist.

3. Aptamer zur Verwendung gemäß Anspruch 1, wobei der Zustand, der mit einer unerwünschten Thrombinaktivität assoziiert ist, Thrombophlebitis, Thrombose im Arteriensystem, Thrombose in der koronaren Zirkulation, Myocardinfarkt, zerebrale Thrombose, zerebraler Schlaganfall, disseminierte-intravasale-Koagulopathie-Syndrom ist.

4. Aptamer zur Verwendung gemäß einem der Ansprüche 1-3, wobei das Aptamer zusammen mit anderen Verbindungen oder parallel zu einer anderen Behandlung zu verabreichen ist.

5. Aptamer zur Verwendung gemäß Anspruch 4, wobei die andere Behandlung eine Operation ist.

6. Aptamer zur Verwendung gemäß Anspruch 4, wobei die anderen Verbindungen gleichzeitig zur Verabreichung des Aptamers, parallel zur Verabreichung des Aptamers verabreicht werden können, oder wobei die Verbindungen und das Aptamer in unterschiedlichen Zeitintervallen verabreicht werden können.

7. Aptamer zur Verwendung gemäß Anspruch 4, wobei das Aptamer und die anderen Verbindungen als ein Teil einer einzelnen Zusammensetzung verabreicht werden können.

8. Zwei-Quadruplex-DNA-Aptamer zur Verwendung beim Vorbeugen und Behandeln eines Zustands, der mit einer unerwünschten Thrombinaktivität assoziiert ist, wobei das Aptamer ein Oligodesoxyribonukleotid ist, mit einer Nukleotidsequenz der allgemeinen Formel:
GGTTGGTGTGGNTGG-Tx-GGTTGGTGTGGNTGG;
worin Tx eine oder mehr T-Basen ist.

## Revendications

1. Aptamère d'ADN quadruplex double pour une utilisation dans une méthode de prévention ou de traitement d'une condition associée à une activité de thrombine indésirable, dans lequel l'aptamère est un oligodésoxyribonucléotide avec une séquence nucléotidique de formule générale :
**GGTTGGTGTGGNTGG-Tx-GGTTGGTGTGGNTGG**
dans laquelle Tx est une ou plusieurs bases T.

2. Aptamère pour une utilisation selon la revendication 1, dans lequel la condition associée à une activité de thrombine indésirable est une thrombose.

3. Aptamère pour une utilisation selon la revendication 1, dans lequel la condition associée à une activité de thrombine indésirable est la thrombophlébite, la thrombose dans le système artériel, la thrombose dans la circulation coronarienne, l'infarctus du myocarde, la thrombose cérébrale, l'accident cérébral, le syndrome de coagulation intravasculaire disséminée.

4. Aptamère pour une utilisation selon l'une quelconque des revendications 1 à 3, dans lequel l'aptamère est administré conjointement avec d'autres composés ou en parallèle avec un autre traitement.

5. Aptamère pour une utilisation selon la revendication 4, dans lequel l'autre traitement est une opération chirurgicale.

6. Aptamère pour une utilisation selon la revendication 4, dans lequel les autres composés peuvent être administrés simultanément avec l'administration de l'aptamère, en parallèle avec l'administration de l'aptamère, ou dans lequel les composés et l'aptamère peuvent être administrés à différents intervalles de temps.

7. Aptamère pour une utilisation selon la revendication 4, dans lequel l'aptamère et les autres composés peuvent être administrés en tant que partie d'une composition unique.

8. Aptamère d'ADN quadruplex double pour une utilisation dans la prévention ou le traitement d'une condition associée à une activité de thrombine indésirable, dans lequel l'aptamère est un oligodésoxyribonucléotide avec une séquence nucléotidique de formule générale :
**GGTTGGTGTGGNTGG-Tx-GGTTGGTGTGGNTGG**
dans laquelle Tx est une ou plusieurs bases T.
